# EUROPEAN PATENT SPECIFICATION

(11) **EP 3 162 403 B1**
(45) Date of publication and mention of the grant of the patent: **03.03.2021**
(21) Application number: 17151099.3
(22) Date of filing: 12.01.2017
(51) Int. Cl.: A24F 40/00, A61M 15/06, H05B 3/40

(54) **ATOMIZER AND ELECTRONIC CIGARETTE HAVING SAME**
ZERSTÄUBER UND ELEKTRONISCHE ZIGARETTE DAMIT
DISPOSITIF D'ATOMISATION ET CIGARETTE ÉLECTRONIQUE COMPORTANT CELUI-CI

(30) Priority: 16.01.2016 CN 201620038520 U; 26.09.2016 CN 201621077045 U
(43) Date of publication of application: 03.05.2017
(73) Proprietor: Shenzhen First Union Technology Co., Ltd., Shenzhen, Guangdong 518104 (CN)
(72) Inventor: LI, Yonghai, Shenzhen, Guangdong 518104 (CN); XU, Zhongli, Shenzhen, Guangdong 518104 (CN); DAI, Changzheng, Shenzhen, Guangdong 518104 (CN); ZOU, Xingbing, Shenzhen, Guangdong 518104 (CN)
(74) Representative: Proi World Intellectual Property GmbH

(56) References cited:
- EP-A1- 2 888 963
- EP-A1- 2 946 678
- EP-A2- 3 138 426
- WO-A1-2016/165057
- CN-A- 104 432 542
- CN-A- 104 970 444
- CN-A- 105 212 270
- CN-A- 105 212 278
- CN-U- 204 317 492
- CN-U- 204 466 907
- CN-U- 204 888 728
- CN-U- 204 907 918
- CN-U- 205 196 999
- CN-U- 205 456 055
- US-A1- 2014 360 514
- US-A1- 2015 144 147

## Description

### TECHNICAL FIELD

The present invention relates to electronic cigarettes, and particularly to an atomizer and an electronic cigarette using same.

### BACKGROUND ART

A typical electronic cigarette includes an atomizer and a power supply. The atomizer includes an atomizing core and a liquid chamber configured for storing tobacco liquid. The atomizing core includes a liquid conducting element and a heating element. The atomizing core is usually replaceable. However, when replacing the atomizing core, tobacco liquid in the liquid chamber may leak out, thus rendering user experience unsatisfactory.

What are needed, therefore, are an atomizer and an electronic cigarette using same, which can overcome the above shortcomings

The document EP 3 138 426 A2 is state of the art according to Article 54(3) EPC, and discloses an atomizer for an electronic cigarette. The atomizer includes a housing, a liquid chamber, an atomizing unit, and a deformable valve. The liquid chamber is in the housing and configured for storing tobacco liquid. The liquid chamber has a liquid injecting opening. The atomizing unit is configured for absorbing and atomizing the tobacco liquid. The deformable valve is arranged in the liquid injecting opening. The valve is capable of being pushed open by an external injector, so that the tobacco liquid can be injected into the liquid chamber. The valve is capable of restoring to its original shape to seal the liquid injecting opening when the injector is removed.

The document CN 105212270 A discloses an atomizer for an electronic cigarette. The atomizer comprises a main body of housing defining a liquid chamber configured for storing tobacco liquid. A mouthpiece is connected with an end of the main body of housing. An atomizing core is arranged in the main body of housing. An atomizing cover is arranged in the liquid chamber, the atomizing core being arranged in the atomizing cover. The atomizing cover has at least one liquid inlet. The atomizing core comprises a heating element and a liquid conducting element surrounding the heating element, the liquid conducting element being configured for absorbing tobacco liquid flowed from the at least one liquid inlet, and the heating element being configured for atomizing the tobacco liquid in the liquid conducting element.

### SUMMARY

The present invention provides an atomizer as defined in claim 1, and an electronic cigarette as defined in claim 11. Further embodiments of the present invention are the subject-matter of the dependent claims.

An exemplary atomizer includes a main body of housing, a mouthpiece, an atomizing core, an atomizing cover. The main body of housing defines a liquid chamber configured for storing tobacco liquid. The mouthpiece is detachably connected with an end of the main body of housing. The atomizing core arranged in the main body of housing, and is detachably connected with the main body of housing. The atomizing cover is arranged in the liquid chamber. The atomizing core is arranged in the atomizing cover. The atomizing cover has a top surface, and the top surface defines at least one liquid inlet. The atomizing core includes a heating element and a liquid conducting element surrounding the heating element. The liquid conducting element is configured for absorbing tobacco liquid flowed from the at least one liquid inlet, and the heating element is configured for atomizing the tobacco liquid in the liquid conducting element.

### BRIEF DESCRIPTION OF THE DRAWINGS

Many aspects of the present disclosure can be better understood with reference to the following drawings. The components in the drawings are not necessarily drawn to scale, the emphasis instead being placed upon clearly illustrating the principles of the present disclosure. Moreover, in the drawings, like reference numerals designate corresponding parts throughout the several views.
FIG. 1 is a cross-sectional view of an atomizer according to a first embodiment.
FIG. 2 is a perspective view of an atomizing core and an atomizing cover of FIG. 1 when separated from each other.
FIG. 3 is a cross-sectional view of the atomizer of FIG. 1 in an inverted state when the atomizing core is taken out.
FIG. 4 is a cross-sectional view of the atomizer of FIG. 1 when a mouthpiece is detached from the main body of housing.
FIG. 5 is an assembled perspective view of an atomizing core and an atomizing cover according to a second embodiment.
FIG. 6 is a perspective view of an electronic cigarette according to a third embodiment.

### DETAILED DESCRIPTION

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, it will be understood by those of ordinary skill in the art that the embodiments described herein can be practiced without these specific details. In other instances, methods, procedures and components have not been described in detail so as not to obscure the related relevant feature being described. Also, the description is not to be considered as limiting the scope of the embodiments described herein. The drawings are not necessarily to scale and the proportions of certain parts have been exaggerated to better illustrate details and features of the present disclosure.

The disclosure is illustrated by way of example and not by way of limitation in the figures of the accompanying drawings in which like references indicate similar elements. It should be noted that references to "an" or "one" embodiment in this disclosure are not necessarily to the same embodiment, and such references mean at least one.

Several definitions that apply throughout this disclosure will now be presented.

The term "outside" refers to a region that is beyond the outermost confines of a physical object. The term "inside" indicates that at least a portion of a region is partially contained within a boundary formed by the object. The term "substantially" is defined to be essentially conforming to the particular dimension, shape or other word that substantially modifies, such that the component need not be exact. For example, substantially cylindrical means that the object resembles a cylinder, but can have one or more deviations from a true cylinder. The term "comprising," when utilized, means "including, but not necessarily limited to"; it specifically indicates open-ended inclusion or membership in the so-described combination, group, series and the like.

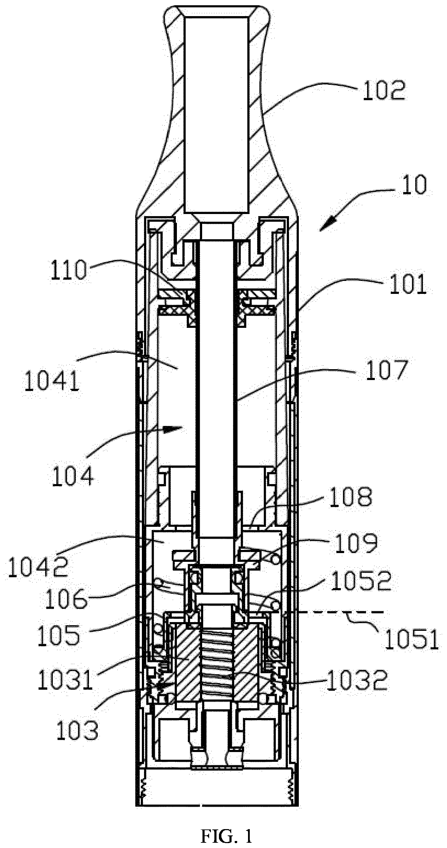

Referring to Fig. 1, an atomizer 10 is shown. The atomizer 10 includes a main body of housing 101, and a mouthpiece 102 detachably connected to an end of the housing 101, and an atomizing core 103 in the main body of housing 101. The main body of housing 101 includes a liquid chamber 104 configured (i.e., structured and arranged) for storing tobacco liquid. The main body of housing 101 includes a housing and a shell of a liquid cup in the housing, and the liquid chamber 104 is defined in the shell of the liquid cup. An atomizing cover 105 is provided in the liquid chamber 104, and is positioned at a bottom part of the liquid chamber 104. The atomizing core 103 is arranged in the atomizing cover 105. An air pipe 107 is further provided in the liquid chamber 104, and two opposite ends of the air pipe are connected to the mouthpiece 102 and the atomizing cover 105, respectively. The atomizing cover 105 includes a top surface 1051 defining at least one liquid inlet 1052. The atomizing core 103 includes a heating element 1032 and a liquid conducting element 1031 surrounding the heating element 1032. The liquid conducting element 1031 is configured for absorbing tobacco liquid, which flows in from the liquid inlet 1052. The heating element 1032 heats the tobacco liquid to form aerosol, the aerosol passes through the air pipe 107 and reaches the mouthpiece 102.

Quite usefully, the liquid conducting element 1031 is an annular hollow structure, and the heating element 1032 is in contact with an internal wall of the liquid conducting element 1031. The liquid conducting element 1031 and the heating element 1032 are oriented along an axial direction of the atomizer 10. An internal space of the liquid conducting element 1031 is in communication with the air pipe 107. A top part of the liquid conducting element 1031 is adjacent to the liquid inlet 1052, the tobacco liquid permeates from the top part to a bottom part, and from an external wall to the internal wall. In the present embodiment, the liquid conducting element 1031 may be made of fiber cotton, or micro porous ceramic, and the heating element is a spiral heating wire.

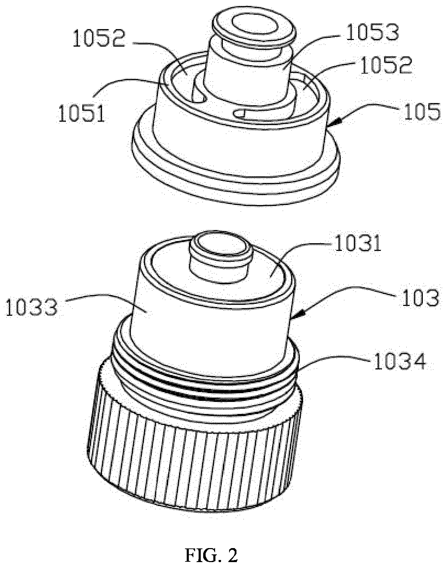

Referring to FIG. 2, the atomizing core 103 includes a holder 1033 configured for supporting the liquid conducting element 1031 and the heating element 1032. The holder 1033 includes a plurality of screw threads 1034 configured for connecting with the main body of housing 101. When the holder 1033 and the main body of housing 101 are connected with each other, the liquid conducting element 1031 and the heating element 1032 are kept inside the atomizing cover 105. It is to be understood that the holder 1033 can be connected directly with a bottom part of the atomizing cover 105. An electrode is further provided at a bottom part of the holder 1033, and the electrode is configured for connecting a power supply.

The atomizing cover 105 defines an atomizing chamber, the atomizing core 103 is received in the atomizing chamber, and the liquid inlet is defined in a top surface 1051 of the atomizing cover 105. In the present embodiment, the atomizing cover 105 defines two symmetric liquid inlets 1052. The atomizing cover 105 further includes a tubular connecting end 1053 extending axially from the top surface 1051 of the atomizing cover 105. The tubular connecting end 1053 is hermetically coupled with a bottom end of the air pipe 107. A bottom end of the atomizing cover 105 is hermetically coupled to the main body of housing 101 via a sealing ring, preventing tobacco liquid in the liquid chamber 104 from leaking into the atomizing chamber via other positions except the liquid inlet 1052.

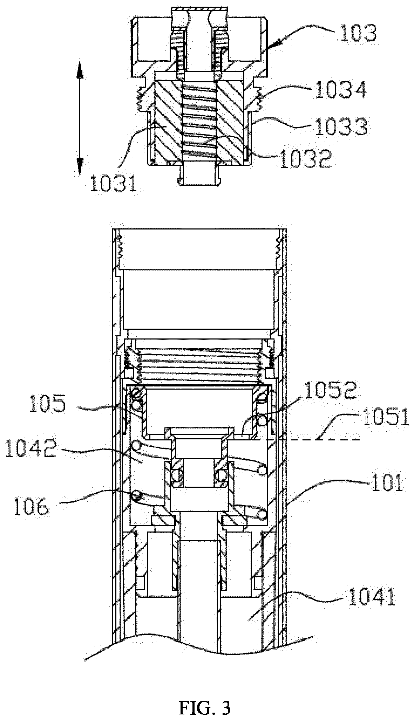

Referring to FIG. 3, when replacing the atomizing core 103, the atomizer 10 is first inverted, the atomizing core 103 is detached from the main body of housing 101, and is then taken out from the atomizing cover 105. In this position, a liquid level of the tobacco liquid in the liquid chamber 104 is usually lower than or coplanar with the top surface 1051 of the atomizing cover 105, avoiding tobacco liquid leakage from the atomizing cover 105.

Referring to FIG. 1 again, an isolation wall is provided in the liquid chamber 104, and the liquid chamber 104 is divided into a first liquid chamber 1041 and a second liquid chamber 1042 by the isolation wall. The isolation wall defines a through hole, the air pipe 107 passes through the through hole. The first liquid chamber 1041 and the second liquid chamber 1042 are arranged from top to bottom along an axial direction. A liquid hole 108 is defined between the air pipe 107 and the isolation wall. The atomizing cover 105 and the atomizing core 103 are arranged in the second liquid chamber 1042. When the liquid hole 108 is not obstructed, the first liquid chamber 1041 is in liquid communication with the second liquid chamber 1042.

Quite usefully, the air pipe 107 is movable along an axial direction, a top end of the air pipe 107 is connected to the mouthpiece 102, and a bottom end of the air pipe 107 is connected to a sealing part 109. The sealing part 109 is configured for sealing the liquid inlet 108. A bottom part of the sealing part 109 abuts against an elastic element. In the present embodiment, the elastic element 106 is a spring. The sealing part 109 includes a flange, a sealing gasket is provided on the flange. When the sealing part 109 is moved upwards, the flange and the sealing gasket seal the liquid hole 108, and the spring abuts against a bottom surface of the flange. Referring to FIG. 1, when the mouthpiece 102 is assembled to the main body of housing 101, the mouthpiece 102 pushes the air pipe 107 and the sealing part 109 to move downwards, the elastic element 106 is compressed, and the liquid hole 108 is opened. In this position, the tobacco liquid in the first liquid chamber 1041 flows into the second liquid chamber 1042, so that the atomizing core 103 can absorb tobacco liquid from the second liquid chamber 1042.

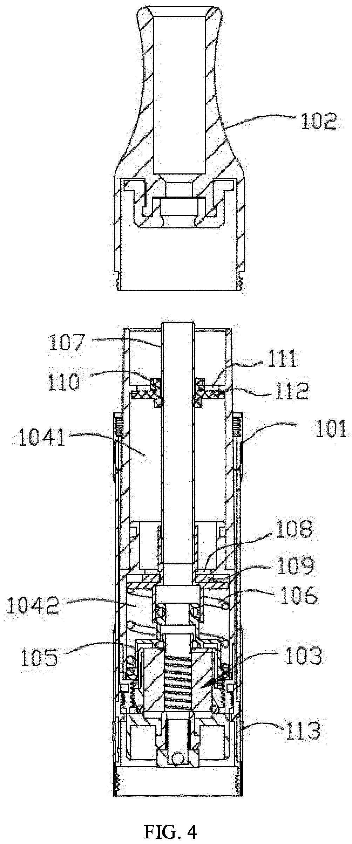

Referring to FIG. 4, when the mouthpiece 102 is detached, the air pipe 107 is driven to move upwards by the elastic element 106, and the sealing part 109 abuts against a bottom surface of the isolation wall and seals the liquid inlet 108. In this position, tobacco liquid in the first liquid chamber 1041 cannot flow into the second liquid chamber 1042. The main body of housing 101 includes a positioning part 110 inside adjacent to the mouthpiece. The positioning part 110 is configured for guiding the air pipe 107 to move axially. A bottom part of the air pipe 107 is connected to the tubular connecting part 1053. The positioning part 110 and the main body of housing 101 cooperatively define a liquid injecting hole 111, which is in communication with the first liquid chamber 1041. In liquid injecting progress, the mouthpiece 102 is detached, and then tobacco liquid can be injected into the first liquid chamber 1041 via the liquid injecting hole 111. In this state, the first liquid chamber 1041 is isolated from the second liquid chamber 1042, thus avoiding liquid leakage from the atomizing core in the second liquid chamber 1042.

Quite usefully, a sealing piece 112 is further provided on the positioning part 110. The sealing piece 112 is configured for sealing the liquid injecting hole 111, and is deformable. The sealing piece 112 is made of silicone, and is circular. The sealing piece 112 nests the air pipe 107. During liquid injecting process, an injector can push the sealing piece 112 open and insert into the first liquid chamber 1041. After finishing injecting, the sealing piece 112 restores to its original shape and seals the liquid injecting hole 111. Accordingly, liquid leakage is prevented when the atomizer 10 is turned over during liquid injecting process.

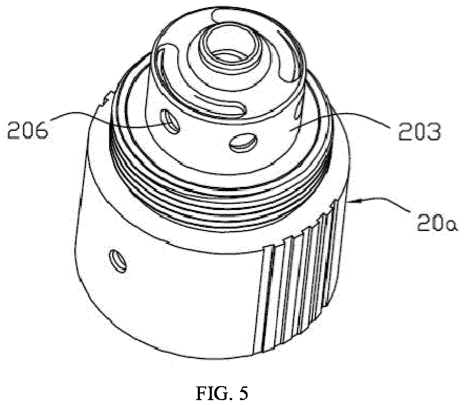

Referring to FIG. 5, an atomizing core 20a and an atomizing cover 203 according to a second embodiment are shown. The atomizing core 20a and the atomizing cover 203 are substantially similar to those of the first embodiment, except that the atomizing cover 203 further defines a plurality of openings 206 in a sidewall. The openings 206 are in communication with an internal space of the atomizing cover 203, so that tobacco liquid can flow into the atomizing core 20a from different directions. Accordingly, the tobacco liquid can flow into the atomizing core 20a more smoothly. It is to be noted that in the atomizer 10 of the first embodiment, the atomizing cover 105 and the atomizing core 103 may be replaced by the atomizing cover 203 and the atomizing core 20a in accordance with the present embodiment.

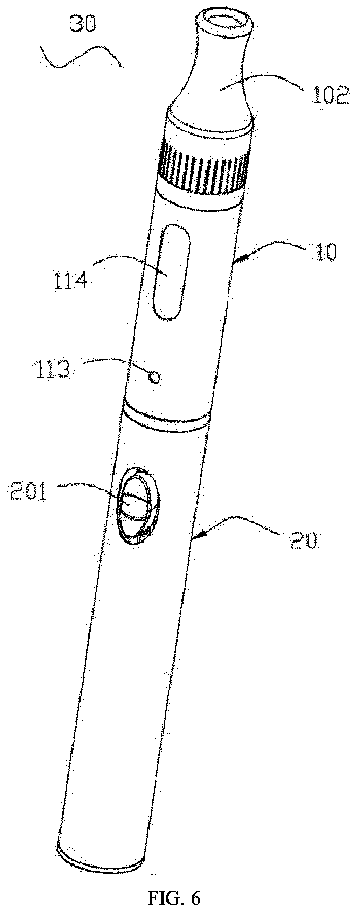

Referring to FIG. 6, an electronic cigarette 30 is shown. The electronic cigarette 30 includes the above-described atomizer 10 and a power supply 20 detachably connected with the atomizer 10. The power supply 20 includes a button 201 configured for turning on/off the atomizer 10. The atomizer 10 defines an air inlet 113, so that external air can enter the atomizer 10 via the air inlet 113.

It is understood that the above-described embodiments are intended to illustrate rather than limit the disclosure. Variations may be made to the embodiments and methods without departing from the scope of the disclosure. Accordingly, it is appropriate that the appended claims be construed broadly and in a manner consistent with the scope of the disclosure.

## Claims

1. An atomizer (10) for an electronic cigarette (30), comprising:
a main body of housing (101) defining a liquid chamber (104) configured for storing tobacco liquid;
a mouthpiece (102) detachably connected with an end of the main body of housing;
an atomizing core (103) arranged in the main body of housing, the atomizing core detachably connected with the main body of housing;
an atomizing cover (105) arranged in the liquid chamber, the atomizing core being arranged in the atomizing cover, the atomizing cover having a top surface (1051), the top surface defining at least one liquid inlet (1052);
the atomizing core comprising a heating element (1032) and a liquid conducting element (1031) surrounding the heating element, the liquid conducting element (1031) being configured for absorbing tobacco liquid flowed from the at least one liquid inlet, and the heating element (1032)
being configured for atomizing the tobacco liquid in the liquid conducting element.

2. The atomizer according to claim 1, wherein the liquid conducting element (1031) is an annular hollow structure, the heating element (1032) contacts with an inner wall of the liquid conducting element; the liquid conducting element (1031) and the heating element (1032) are arranged along an axial direction of the atomizer.

3. The atomizer according to claim 2, wherein the liquid conducting element (1031) is made of fiber cotton or micro porous ceramic, and the heating element (1032) comprises a spiral heating wire.

4. The atomizer according to claim 1, wherein the atomizing core (103) further comprises a holder (1033) configured for supporting the liquid conducting element and the heating element, and keeping the liquid conducting element (1031) and the heating element (1032) in the atomizing cover; the holder (1033) is detachable connected with the main body of housing.

5. The atomizer according to claim 1, wherein the liquid chamber (104) is divided into a first liquid chamber (1041) and a second liquid chamber (1042), the atomizer further comprises a liquid hole (108) defined between the first liquid chamber and the second liquid chamber, and the atomizing cover (105) and the atomizing core (103) are arranged in the second liquid chamber.

6. The atomizer according to claim 5, further comprising an air pipe (107) in the liquid chamber (104), a sealing part (109), an elastic element (106), wherein the air pipe is movable along an axial direction thereof, a top end of the air pipe is connected with the mouthpiece, a bottom end of the air pipe is connected with the sealing part, the sealing part (109) is configured for sealing the liquid hole (108), the elastic element (106) abuts against a bottom part of the sealing part; when the mouthpiece is assembled to the main body of housing, the mouthpiece (102) pushes the air pipe (107) to move downwards, so that the sealing part (109) opens the liquid hole; when the mouthpiece is detached, the air pipe is driven by the elastic element to move upwards, so that the sealing part seals the liquid hole.

7. The atomizer according to claim 6, wherein the main body of housing (101) comprises a positioning part (110) adjacent to the mouthpiece, the positioning part is configured for guiding the air pipe (107) to move axially, the positioning part and the main body of housing cooperatively define a liquid injecting hole (111), and the liquid injecting hole is in communication with the first liquid chamber (1041).

8. The atomizer according to claim 7, further comprising a sealing piece (112) provided on the positioning part, wherein the sealing piece (112) is configured for sealing the liquid injecting hole, and is deformable.

9. The atomizer according to claim 6, wherein the atomizing cover (105) comprises a tubular connecting end (1053) on the top surface (1051), and the tubular connecting end (1053) is hermetically coupled with the bottom end of the air pipe (107).

10. The atomizer according to claim 1, wherein the atomizing cover (203) further comprises a sidewall defining a plurality of openings (206).

11. An electronic cigarette (30) comprising:
an atomizer (10) according to any of claims 1-10; and
a power supply (20) configured for supplying the atomizer power.

## Patentansprüche

1. Zerstäuber (10) für eine elektronische Zigarette (30), umfassend:
einen Gehäusehauptkörper (101), der eine Flüssigkeitskammer (104) definiert, die zum Speichern von Tabakflüssigkeit konfiguriert ist;
ein Mundstück (102), das abnehmbar mit einem Ende des Gehäusehauptkörpers verbunden ist;
einen Zerstäuberkern (103), der in dem Gehäusehauptkörper angeordnet ist, wobei der Zerstäuberkern abnehmbar mit dem Gehäusehauptkörper verbunden ist;
eine Zerstäuberabdeckung (105), die in der Flüssigkeitskammer angeordnet ist,
wobei der Zerstäuberkern in der Zerstäuberabdeckung angeordnet ist, wobei die Zerstäuberabdeckung eine obere Fläche (1051) aufweist, wobei die obere Fläche mindestens einen Flüssigkeitseinlass (1052) definiert;
der Zerstäubungskern ein Heizelement (1032) und ein flüssigkeitsleitendes Element (1031) umfasst, das das Heizelement umgibt, wobei das flüssigkeitsleitende Element (1031) zum Absorbieren von Tabakflüssigkeit konfiguriert ist, die von dem mindestens einen Flüssigkeitseinlass strömt, und das Heizelement (1032) zum Zerstäuben der Tabakflüssigkeit in dem flüssigkeitsleitenden Element konfiguriert ist.

2. Zerstäuber nach Anspruch 1, wobei das flüssigkeitsleitende Element (1031) eine ringförmige Hohlstruktur ist, das Heizelement (1032) mit einer Innenwand des flüssigkeitsleitenden Elements in Kontakt steht; das flüssigkeitsleitende Element (1031) und das Heizelement (1032) entlang einer axialen Richtung des Zerstäubers angeordnet sind.

3. Zerstäuber nach Anspruch 2, wobei das flüssigkeitsleitende Element (1031) aus Baumwollfasern oder mikroporöser Keramik hergestellt ist und das Heizelement (1032) einen spiralförmigen Heizdraht umfasst.

4. Zerstäuber nach Anspruch 1, wobei der Zerstäuberkern (103) ferner einen Halter (1033) umfasst, der zum Tragen des flüssigkeitsleitenden Elements und des Heizelements konfiguriert ist,
und das flüssigkeitsleitende Element (1031) und das Heizelement (1032) in der Zerstäuberabdeckung gehalten sind; der Halter (1033) ist abnehmbar mit dem Hauptkörper des Gehäuses verbunden.

5. Zerstäuber nach Anspruch 1, wobei die Flüssigkeitskammer (104) in eine erste Flüssigkeitskammer (1041) und eine zweite Flüssigkeitskammer (1042) unterteilt ist, der Zerstäuber ferner ein Flüssigkeitsloch (108) umfasst, das zwischen der ersten Flüssigkeitskammer und der zweiten Flüssigkeitskammer definiert ist, und die Zerstäuberabdeckung (105) und der Zerstäuberkern (103) in der zweiten Flüssigkeitskammer angeordnet sind.

6. Zerstäuber nach Anspruch 5, ferner umfassend ein Luftrohr (107) in der Flüssigkeitskammer (104), ein Dichtungsteil (109), ein elastisches Element (106), wobei das Luftrohr entlang einer axialen Richtung desselben bewegbar ist, ein oberes Ende des Luftrohrs mit dem Mundstück verbunden ist, ein unteres Ende des Luftrohrs mit dem Dichtungsteil verbunden ist, das Dichtungsteil (109) zum Abdichten des Flüssigkeitslochs (108) konfiguriert ist, das elastische Element (106) gegen einen unteren Teil des Dichtungsteils anliegt; wenn das Mundstück mit dem Hauptkörper des Gehäuses zusammengebaut ist, drückt das Mundstück (102) das Luftrohr (107), um sich nach unten zu bewegen, so dass das Dichtungsteil (109) das Flüssigkeitsloch öffnet; wenn das Mundstück abgenommen wird, wird das Luftrohr durch das elastische Element angetrieben, um sich nach oben zu bewegen, so dass das Dichtungsteil das Flüssigkeitsloch abdichtet.

7. Zerstäuber nach Anspruch 6, wobei der Hauptkörper des Gehäuses (101) ein Positionierungsteil (110) angrenzend an das Mundstück umfasst, das Positionierungsteil so konfiguriert ist, dass es das Luftrohr (107) so führt, dass es sich axial bewegt, das Positionierungsteil und der Hauptkörper des Gehäuses zusammen ein Flüssigkeitseinspritzloch (111) definieren und das Flüssigkeitseinspritzloch in Verbindung mit der ersten Flüssigkeitskammer (1041) steht.

8. Zerstäuber nach Anspruch 7, ferner umfassend ein Dichtungsstück (112), das an dem Positionierungsteil vorgesehen ist, wobei das Dichtungsstück (112) zum Abdichten des Flüssigkeitseinspritzlochs konfiguriert ist und verformbar ist.

9. Zerstäuber nach Anspruch 6, wobei die Zerstäuberabdeckung (105) ein rohrförmiges Verbindungsende (1053) an der oberen Fläche (1051) aufweist und das rohrförmige Verbindungsende (1053) hermetisch mit dem unteren Ende des Luftrohrs (107) gekoppelt ist.

10. Zerstäuber nach Anspruch 1, wobei die Zerstäuberabdeckung (203) ferner eine Seitenwand aufweist, die eine Vielzahl von Öffnungen (206) definiert.

11. Elektronische Zigarette (30), umfassend:
einen Zerstäuber (10) nach einem der Ansprüche 1-10; und
eine Stromversorgung (20), die zum Versorgen des Zerstäubers mit Energie konfiguriert ist.

## Revendications

1. Un atomiseur (10) pour une cigarette électronique (30), comprenant :
un corps principal de boîtier (101) définissant une chambre de liquide (104) configurée pour stocker du liquide de tabac ;
un embout (102) relié de manière amovible à une extrémité du corps principal du logement ;
un noyau de pulvérisation (103) disposé dans le corps principal du boîtier, le noyau de pulvérisation étant relié de manière amovible au corps principal du boîtier ;
un couvercle de pulvérisation (105) disposé dans la chambre de liquide, le noyau de pulvérisation étant disposé dans le couvercle de pulvérisation, le couvercle de pulvérisation ayant une surface supérieure (1051), la surface supérieure définissant au moins une entrée de liquide (1052) ;
le noyau d'atomisation comprenant un élément chauffant (1032) et un élément conducteur de liquide (1031) entourant l'élément chauffant, l'élément conducteur de liquide (1031) étant configuré pour absorber le liquide de tabac s'écoulant de la au moins une entrée de liquide, et l'élément chauffant (1032) étant configuré pour atomiser le liquide de tabac dans l'élément conducteur de liquide.

2. L'atomiseur selon la revendication 1, dans lequel l'élément conducteur de liquide (1031) est une structure annulaire creuse, l'élément chauffant (1032) est en contact avec une paroi intérieure de l'élément conducteur de liquide ; l'élément conducteur de liquide (1031) et l'élément chauffant (1032) sont disposés le long d'une direction axiale du pulvérisateur.

3. L'atomiseur selon la revendication 2, dans lequel l'élément conducteur de liquide (1031) est fait de fibre de coton ou de céramique microporeuse, et l'élément chauffant (1032) comprend un fil chauffant en spirale.

4. L'atomiseur selon la revendication 1, dans lequel le noyau d'atomisation (103) comprend en outre un support (1033) configuré pour supporter l'élément conducteur de liquide et l'élément chauffant,
et en maintenant l'élément conducteur de liquide (1031) et l'élément chauffant (1032) dans le couvercle de pulvérisation ; le support (1033) est relié de manière amovible au corps principal du boîtier.

5. L'atomiseur selon la revendication 1, dans lequel la chambre de liquide (104) est divisée en une première chambre de liquide (1041) et une seconde chambre de liquide (1042), l'atomiseur comprend en outre un trou de liquide (108) défini entre la première chambre de liquide et la seconde chambre de liquide, et le couvercle d'atomisation (105) et le noyau d'atomisation (103) sont disposés dans la seconde chambre de liquide.

6. L'atomiseur selon la revendication 5, comprenant en outre un tuyau d'air (107) dans la chambre de liquide (104), une partie d'étanchéité (109), un élément élastique (106), dans lequel le tuyau d'air est mobile le long d'une direction axiale de celui-ci, une extrémité supérieure du tuyau d'air est reliée à l'embout, une extrémité inférieure du tuyau d'air est reliée à la partie d'étanchéité, la partie d'étanchéité (109) est configurée pour sceller le trou de liquide (108), l'élément élastique (106) vient en butée contre une partie inférieure de la partie d'étanchéité ; lorsque l'embout est assemblé au corps principal du boîtier, l'embout (102) pousse le tuyau d'air (107) pour qu'il se déplace vers le bas, de sorte que la partie d'étanchéité (109) ouvre le trou de liquide ; lorsque l'embout est détaché, le tuyau d'air est entraîné par l'élément élastique pour se déplacer vers le haut, de sorte que la partie d'étanchéité ferme le trou de liquide.

7. L'atomiseur selon la revendication 6, dans lequel le corps principal du boîtier (101) comprend une partie de positionnement (110) adjacente à l'embout, la partie de positionnement est configurée pour guider le tuyau d'air (107) afin qu'il se déplace axialement, la partie de positionnement et le corps principal du boîtier définissent conjointement un trou d'injection de liquide (111), et le trou d'injection de liquide est en communication avec la première chambre de liquide (1041).

8. L'atomiseur selon la revendication 7, comprenant en outre une pièce d'étanchéité (112) prévue sur la partie de positionnement, dans lequel la pièce d'étanchéité (112) est configurée pour sceller le trou d'injection de liquide, et est déformable.

9. L'atomiseur selon la revendication 6, dans lequel le couvercle de pulvérisation (105) comprend une extrémité de raccordement tubulaire (1053) sur la surface supérieure (1051), et l'extrémité de raccordement tubulaire (1053) est couplée hermétiquement avec l'extrémité inférieure du tuyau d'air (107).

10. L'atomiseur selon la revendication 1, dans lequel le couvercle de pulvérisation (203) comprend en outre une paroi latérale définissant une pluralité d'ouvertures (206).

11. Une cigarette électronique (30) comprenant :
un atomiseur (10) selon l'une quelconque des revendications 1 à 10 ; et
une alimentation électrique (20) configurée pour fournir la puissance de l'atomiseur.
